# EUROPÄISCHE PATENTSCHRIFT

(11) **EP 1 203 944 B1**
(45) Veröffentlichungstag und Bekanntmachung des Hinweises auf die Patenterteilung: **01.03.2006**
(21) Anmeldenummer: 00124107.4
(22) Anmeldetag: 06.11.2000
(51) Int. Cl.: G01N 21/64, A61K 41/00, A61K 49/00

(54) **Tumorzellenidentifizierungsverfahren**
Tumor cell identification method
Procédé d'identification de cellules turmorales

(43) Veröffentlichungstag der Anmeldung: 08.05.2002
(73) Patentinhaber: Hofstetter, Alfons, Prof.Dr.med., 82008 Unterhaching (DE); Tauber, Stephan, Dr. med., 80803 München (DE)
(72) Erfinder: Hofstetter, Alfons, Prof.Dr.med., 82008 Unterhaching (DE); Tauber, Stephan, Dr. med., 80803 München (DE)
(74) Vertreter: Hofstetter, Alfons J.

(56) Entgegenhaltungen:
- WO-A-96/39188
- WO-A-97/08523
- US-A- 3 973 129
- US-A- 4 608 990
- US-A- 5 411 891
- US-A- 5 955 490
- US-A- 6 034 267
- DE ROSA F S ET AL: "A vehicle for photodynamic therapy of skin cancer: influence of dimethylsulphoxide on 5-aminolevulinic acid in vitro cutaneous permeation and in vivo protoporphyrin IX accumulation determined by confocal microscopy" JOURNAL OF CONTROLLED RELEASE,NL,ELSEVIER SCIENCE PUBLISHERS B.V., AMSTERDAM, Bd. 65, Nr. 3, April 2000 (2000-04), Seiten 359-366, XP004190334 ISSN: 0168-3659

## Beschreibung

Die Erfindung betrifft ein Tumorzellenidentifizierungsverfahren zur automatisierten in vitro Identifizierung von Tumorzellen, in denen Protoporphyrin IX angereichert ist, in einer Flüssigkeit, unter Verwendung einer Tumorzellenidentifizierungsvorrichtung.

Zur Erkennung eines Harnblasenkarzinoms eines Patienten ist eine 5-Aminolävulinsäure-induzierte Fluoreszenzendoskopie bekannt. Bei diesem Untersuchungsverfahren wird einem Patienten zwei bis vier Stunden vor der endoskopischen Untersuchung mit einem Katheter 5-Aminolävulinsäure (5-ALA) in die Harnblase instilliert. Dabei werden 1,5 g 5-ALA in 50ml einer auf pH 4,9 gepufferten Lösung verwendet. Insbesondere in Tumorzellen wird das 5-ALA in Protoporphyrin IX umgewandelt, das bei einer Anregung mit energiereichem, blauviolettem Licht einer Wellenlänge um 400 nm eine charakteristische rötliche Fluoreszenz mit einer Wellenlänge von 630 - 700 nm abgibt. Bei der nachfolgenden endoskopischen Untersuchung wird das gefilterte Licht einer Xenon-Kurzbogenlampe durch ein Beleuchtungsbündel eines Endoskops auf das Harnblasengewebe gerichtet. Zur Filterung des von der Xenon-Kurzbogenlampe erzeugten inkohärenten Lichtes wird eine Filterbeschichtung verwendet, die lediglich den blauen Spektralanteil hindurchläßt. Zur Beobachtung der Fluoreszenz, die durch das Protoporphyrin IX der Tumorzellen emittiert wird, reicht das menschliche Auge. Rückgestreutes Anregungslicht muß jedoch weitgehend weggefiltert werden, wobei der noch transmittierte Restanteil eine optimale Erkennung suspekter Areale in der Harnblase ermöglicht. Mit Hilfe der beschriebenen Fluoreszenzzystoskopie mit 5-ALA können Tumore oft sehr viel früher entdeckt und schließlich behandelt werden, als dies bei Anwendung einer konventionellen Endoskopie mit Weißlicht möglich wäre.

Ähnliche Verfahren werden inzwischen auch zur Früherkennung eines Bronchialkarzinoms, eines malignen Glioms, von Neoplasien in Mundhöhle und Larynx, einer Cervixdysplasie oder einer Endometriose, von Dysplasien bei Colitis Ulcerosa oder auch zur Früherkennung des Barrett-Ösophagus verwendet.

Beispielsweise wird bei Patienten mit Verdacht auf ein Bronchialkarzinom 5-ALA entweder inhalativ (200 bis 250 mg 5-ALA) oder oral (20 bis 40 mg 5-ALA/kg Körpergewicht) verabreicht. Anschließend wird eine Gewebeprobe entnommen und mit Hilfe der Fluoreszenzdiagnostik untersucht. Zu diesem Zweck wird ein Fluoreszenzmikroskop eingesetzt, in dem die Probe mit blauviolettem Licht einer Wellenlänge von etwa 400 nm bestrahlt wird und die durch das Protoporphyrin IX erzeugte Fluoreszenz durch einen professionellen Diagnostiker beobachtet und bewertet wird.

Aus der US 5,955,490 ist bekannt, wie Abnormalien in Körperflüssigkeiten durch induzierte Fluoreszenz detektiert werden können. Die Abnormalien können dabei kanzerogenen Ursprungs sein. Zu diesem Zweck kann einem Patienten ALA oder eine andere Vorstufe des Protoporphyrins IX appliziert werden, bevor ihm eine Körperflüssigkeit, insbesondere Blut, entnommen wird. Alternativ kann die Induktion der Biosynthese des Protoporphyrins IX auch in vitro durch Zugabe von ALA oder einer anderen Vorstufe von Porphyrinen erreicht werden. Die Detektion von Zellen, in denen Protoporphyrin IX oder ein anderes Porphyrin angereichert ist, erfolgt dann beispielsweise durch "Fluorescence Flow Cytometry", Fluoreszenz-Mikroskopie oder quantitative Spektrophotofluorimetrie. Dabei wird eine Bestrahlung mit Licht einer Wellenlänge von 410 nm vorgenommen, wobei gleichzeitig die emittierte Fluoreszenz bei 635 nm gemessen wird.

Nachteilig an den genannten Verfahren ist jedoch, daß stets ein größerer Eingriff vorgenommen werden muß, bei dem ein Endoskop eingeführt bzw. eine Gewebeprobe entnommen werden muß. Dies führt dazu, daß diese Verfahren beispielsweise nicht als Routineuntersuchungen bei tumorgefährdeten Patienten durchgeführt werden können. Außerdem sind größere Gewebsareale oft nur schwer auf diese Weise zu untersuchen. Weiterhin nachteilig ist, daß die genannten Verfahren nur von sehr erfahrenen Ärzten durchgeführt werden können, da die einzelnen Verfahrensschritte sehr viel diagnostische Erfahrung und operative Geschicklichkeit voraussetzen.

Demgemäß ist es Aufgabe der Erfindung, ein Tumorzellenidentifizierungsverfahren bereitzustellen, mit der bzw. mit dem Tumore mit hoher Sicherheit und Zuverlässigkeit erkannt werden können, ohne daß ein großer Eingriff erforderlich ist und ohne daß eine so qualifizierte Arbeitskraft wie ein Arzt benötigt wird, um das Tumorzellenidentifizierungsverfahren zu bedienen bzw. anzuwenden.

Diese Aufgabe wird erfindungsgemäß durch ein Tumorzellenidentifizierungsverfahren zur automatisierten in vitro Identifizierung von Tumorzellen, in denen Protoporphyrin IX angereichert ist, in einer Flüssigkeit, unter Verwendung einer Tumorzellenidentifizierungsvorrichtung gelöst, welches folgende Schritte umfasst: a) Bestrahlen mindestens einer Zelle in der Flüssigkeit mit Licht einer Wellenlänge von im Wesentlichen 400 nm mit der Tumorzellenidentifizierungsvorrichtung, die eine Lichtquelle und ein Sensorelement umfasst, wobei die sich in der Flüssigkeit befindlichen Zellen durch die Lichtquelle gesamtheitlich oder sequentiell bestrahlbar sind; und b) gesamtheitliches oder sequentielles Erfassen der von der mindestens einen Zelle in der Flüssigkeit emittierten Lichtmenge von Licht einer Wellenlänge von im Wesentlichen 630 nm bis 700 nm pro Zeiteinheit durch das Sensorelement und Erzeugen mindestens eines für die erfasste Lichtmenge charakteristischen Signals, wobei die Flüssigkeit innerhalb der Tumoridentifizierungsvorrichtung vor Beendigung von Schritt b) von Fremdlicht abgeschlossen gelagert wird und für das Bestrahlen der Flüssigkeit und das Erfassen der emittierten Lichtmenge gemäß den Verfahrensschritten a) und b) ein Gefäß der Tumoridentifizierungsvorrichtung verwendet wird, wobei das Gefäß in Form einer Schale mit einem flachen Boden ausgebildet ist, in der die Flüssigkeit flächig verteilbar ist und zur Lenkung des Lichts auf diesen Boden mindestens ein Spiegelelement in der Tumoridentifizierungsvorrichtung ausgebildet ist. Durch das erfindungsgemäße Tumorzellenidentifizierungsverfahren wird folglich vermieden, daß durch Fremdlicht eine Ausbleichung erfolgt, die dazu führen würde, daß das Protoporphyrin IX in den Tumorzellen nicht mehr identifiziert werden kann.

Mit Hilfe des erfindungsgemäßen Tumorzellenidentifizierungsverfahrens können alle Flüssigkeiten und Sekrete untersucht werden, die eine größere Anzahl von Zellen aufweisen, die von einem Gewebe stammen, mit dem die Flüssigkeiten oder die Sekrete in Berührung gekommen sind. Ist dieses Gewebe teilweise tumorös, gelangen auch Tumorzellen in die Flüssigkeit, die unter bestimmten Bedingungen mit Protoporphyrin IX angereichert sind oder beispielsweise durch Induktion mit 5-ALA in vitro mit Protoporphyrin IX angereichert werden können. Innerhalb der Tumorzellenidentifizierungsvorrichtung sind die in der Flüssigkeit befindlichen Zellen automatisiert mit der Anregungswellenlänge des Protoporphyrins IX bestrahlbar. Ist unter diesen Zellen mindestens eine Tumorzelle, so emittiert diese aufgrund der Bestrahlung Licht einer Wellenlänge von im Wesentlichen 630 bis 700 nm, die durch das Sensorelement erfaßbar ist. Durch das Sensorelement ist ein für die erfaßte Lichtmenge charakteristisches Signal erzeugbar, das einem Bediener der Tumorzellenidentifizierungsvorrichtung, der eine nur kurz angelemte Arbeitskraft sein kann, ein Ergebnis liefert, das von einem erfahrenen Arzt zu gegebener Zeit interpretiert werden kann. Mit Hilfe des erfindungsgemäßen Tumorzellenidentifizierungsverfahrens kann selbst eine einzige Tumorzelle erkannt werden, was zu einer äußerst frühen Erkennung von Tumoren führt. Da mit dem erfindungsgemäßen Tumorzellenidentifizierungsverfahren Flüssigkeiten wie Körperflüssigkeiten und Sekrete untersucht werden können, sind keine der oben beschriebenen größeren chirurgischen Eingriffe bei dem Patienten erforderlich. Eine detaillierte Diagnostik, durch die die Lage und die Position des Tumors erfaßt werden kann, sollte sich an eine Identifizierung einer Tumorzelle anschließen. Zudem können die gesamten Zellen in der Flüssigkeit gleichzeitig bestrahlt werden, so dass die Identifizierung von Tumorzellen äußerst schnell erfolgen kann. Zu diesem Zweck weist die Tumorzellenidentifizierungsvorrichtung das genannte mindestens eine Spiegelelement auf, durch das das von der Lichtquelle erzeugte Licht auf die Fläche lenkbar ist. Um Teile der Tumorzellenidentifizierungsvorrichtung bzw. die gesamte Tumorzellenidentifizierungsvorrichtung von Fremdlicht abzuschließen, kann das Gefäß und/oder die Lichtquelle und/oder das Sensorelement und/oder das eine Spiegelelement und/oder die Messkammer innerhalb einer Dunkelkammer angeordnet sein.

Bei dem erfindungsgemäßen Tumorzellenidentifizierungsverfahren kann zudem vorgesehen sein, dass die Bewegung der Flüssigkeit bei einem der genannten Verfahrensschritte oder bei beiden Verfahrensschritten durch die Schwerkraft oder eine Pumpe erfolgt.

Das Gefäß kann mindestens in einem Bereich lichtdurchlässig, insbesondere für Licht der Wellenlänge 350 bis 450 nm und/oder von 600 bis 700 nm, sein. Diesen Ansprüchen genügt beispielsweise ein Gefäß aus einem geeigneten Glas, das insbesondere als Glasschale ausgebildet ist.

Das Anregungslicht kann folglich auch durch diesen mindestens einen Bereich des Gefäßes auf die Zellen in der Flüssigkeit gelenkt werden.

Die Lichtquelle kann einen Filter aufweisen, der nur für Licht einer Wellenlänge von im Wesentlichen 400 nm durchlässig ist, wogegen das Sensorelement mindestens einen Filter aufweisen kann, der nur für Licht einer Wellenlänge von im Wesentlichen 630 bis 700 nm durchlässig ist. Durch geeignete Anordnung von Filtern kann die für die Erfindung notwendige Wellenlängenspezifität besonders günstig und einfach erreicht werden.

Eine weitere vorteilhafte Ausgestaltung der Erfindung sieht vor, daß die Tumorzellenidentifizierungsvorrichtung eine Ausgabeeinheit aufweist, durch die das mindestens eine von dem Sensorelement erzeugte Signal verstärkbar und/oder umwandelbar und/oder mit einem vorbestimmten Protoporphyrin-IX-Konzentrationsgrenzwert vergleichbar ist, wobei durch die Ausgabeeinheit ein Ausgabesignal erzeugbar ist, das angibt mit welcher Wahrscheinlichkeit in der Flüssigkeit tumorsuspekte Zellen vorliegen. In dem letztgenannten Fall ist noch nicht einmal eine Interpretation des Ergebnisses durch einen erfahrenen Arzt notwendig, da auch die Auswertung automatisiert durch die Tumorzellenidentifizierungsvorrichtung durchführbar ist.

Die Flüssigkeit kann Zellen einer Spüllösung, die mit einem Gewebe eines Patienten in Berührung gekommen ist, und/oder Zellen einer Körperflüssigkeit, insbesondere Urin und/oder Blut und/oder Speichel und/oder Scheidensekret und/oder Spermienflüssigkeit und/oder Lungenauswurf und/oder Magensaft und/oder Bauchspeicheldrüsenflüssigkeit aufweisen. Als Flüssigkeit kann auch direkt eine solche Spüllösung und/oder eine solche Körperflüssigkeit eingesetzt werden. Die Wahl der Flüssigkeit hängt selbstverständlich davon ab, für welche Organe bzw. für welches Gewebe ein Tumorverdacht besteht. Die in einer Spüllösung oder in einer Körperflüssigkeit befindlichen Zellen können auch durch eine Zentrifugation angereichert werden, so daß eine große Anzahl von Zellen in einem Schritt mit der Tumorzellenidentifizierungsvorrichtung untersucht werden kann. Die durch die Zentrifugation angereicherten Zellen können auch in einer weiteren Flüssigkeit gelöst bzw. aufgenommen werden.

Weiterhin wird durch die Erfindung ein Verfahren zur Identifizierung von Tumorzellen bereitgestellt, das das erfindungsgemäße Tumorzellenidentifizierungsvertahren umfaßt, jedoch zunächst einen Schritt aufweist, in dem die Tumorzellen mit Protoporphyrin IX angereichert werden. Diese Anreicherung kann beispielsweise durch 30- bis 120-minütige Inkubation mit 5-ALA in vitro, insbesondere bei 37°C, geschehen. Auf diese Weise kann eine zu einem beliebigen Zeitpunkt entnommene Flüssigkeit in Bezug auf mögliche Tumorzellen untersucht werden, ohne daß es einer Vorbehandlung des Patienten bedarf. Das Verfahren kann folglich mit jeder routinemäßig entnommenen Flüssigkeit durchgeführt werden und ist somit für prophylaktische Untersuchungen besonders geeignet.

In einer Weiterbildung dieses Verfahrens kann in Tumorzellen Protoporphyrin IX angereichert werden, indem eine Lösung, die 5-ALA aufweist, in einen Patienten instilliert wird, wobei dem Patienten eine oder die Flüssigkeit nach 30 bis 120 Minuten entnommen wird. Da das Instillieren insbesondere auch durch Inhalation oder orale Gabe erfolgen kann, ist auch diese Weiterbildung genau wie die vorher erwähnte Ausgestaltung dazu geeignet, an dem Patienten auch von einer nur kurz eingewiesenen Person durchgeführt zu werden.

Die mindestens eine Zelle kann durch mindestens einen Zentrifugationsschritt konzentriert oder auch pelletiert werden. Pelletierte Zellen können anschließend in einer Flüssigkeit, die sich besonders gut für das Tumoridentifizierungsverfahren eignet, aufgenommen werden.

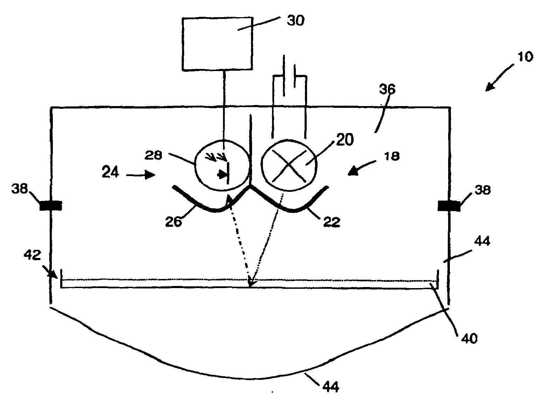

Im Folgenden werden Ausführungsbeispiele der Erfindung unter Hinweis auf die beigefügte Zeichnung näher beschrieben.

Die Figur zeigt dabei eine Tumorzellenidentifizierungsvorrichtung zur Durchführung des erfindungsgemäßen Tumorzellenidentifizierungsverfahrens.

Die Tumorzellenidentifizierungsvorrichtung 10 umfasst eine Dunkelkammer 36. Die Dunkelkammer 36 kann mit Hilfe von Dunkelkammerverschlüssen 38 geöffnet und wieder geschlossen werden, um eine Flüssigkeit 40 in einem Gefäß 42 in die Dunkelkammer 36 einzubringen. Die Flüssigkeit 40, beispielsweise eine Blutprobe oder eine Speichelprobe, ist innerhalb des Gefäßes 42 über eine relativ große Fläche verteilt. Bei dem Gefäß 42 handelt es sich in der hier beschriebenen Ausführungsform um eine Glasschale. Durch eine Lichtquelle 18, bestehend aus einer Xenon-Lampe 20 und einem Filter 22, wird Licht einer Wellenlänge von etwa 400 nm erzeugt, das einerseits direkt auf die Glasschale 42 und damit auf die Flüssigkeit 40 fällt und andererseits durch Spiegel 44, mit denen die Dunkelkammer 36 ausgekleidet ist, auf die Flüssigkeit 40 gelenkt wird.

In der Dunkelkammer 36 befindet sich auch eine äußerst empfindliche Photodiode 28 und ein Filter 26, die gemeinsam ein Sensorelement 24 bilden und selektiv rötliches Licht um 630 nm registrieren, das von den mit Protoporphyrin IX angereicherten Tumorzellen in der Flüssigkeit 40 emittiert wird. Die Ausführungsform weist daher neben den Spiegeln 44 weitere optische Elemente auf, die dazu führen, daß das Fluoreszenzlicht auf das Sensorelement 24 gelenkt wird. Eine elektronische Ausgabeeinheit 30 verstärkt und verwandelt das von dem Sensorelement 24 erzeugte Signal und gibt entsprechend einem einstellbaren Protoporphyrin-IX-Konzentrationsgrenzwert an, ob und/oder mit welcher Wahrscheinlichkeit ein Tumorverdacht besteht. Auch diese Ausführungsform kann sehr kompakt ausgebildet sein und in Form eines portablen Handgerätes hergestellt werden.

Um in möglichen Tumorzellen innerhalb der Flüssigkeit 40 Protoporphyrin IX anzureichern, wird dem zu untersuchenden Patienten eine Lösung mit 5-ALA instilliert und die Flüssigkeit 40 nach ca. 30 Minuten bis zu 2 Stunden gewonnen. Soll, wie in dem hier aufgeführten Beispiel, eine Speichelprobe entnommen werden und sollen auf diese Weise Neoplasien in der Mundhöhle aufgespürt werden, wird 5-ALA in der Mundhöhle als Spüllösung appliziert. Dabei handelt es sich um eine 0,4-prozentige Lösung, mit der 15 Minuten lang mit Unterbrechung gespült wird. Diese Mischung aus Speichelflüssigkeit und Spüllösung kann dann mit der erfindungsgemäßen Tumorzellenidentifizierungsvorrichtung untersucht werden. Die hierfür nötige Betreuung und Anweisung des Patienten kann auch von einer nur kurz angelernten Person durchgeführt werden, ohne daß die Anwesenheit eines erfahrenen Mediziners notwendig ist. Dies gilt gleichermaßen auch für die Bedienung der erfindungsgemäßen Tumorzellenidentifizierungsvorrichtung. Für eine Untersuchung einer Körperflüssigkeit kann eine orale Verabreichung von 5-ALA, wie etwa 10 mg pro kg Körpergewicht, zu einer ausreichenden Anreicherung von Protoporphyrin IX in den Tumorzellen dieser Körperflüssigkeit führen.

## Patentansprüche

1. Tumorzellenidentifizierungsverfahren zur automatisierten in vitro Identifizierung von Tumorzellen, in denen Protoporphyrin IX angereichert ist, in einer Flüssigkeit, unter Verwendung einer Tumorzellenidentifizierungsvorrichtung (10) folgende Schritte umfassend:
a) Bestrahlen mindestens einer Zelle in der Flüssigkeit mit Licht einer Wellenlänge von im Wesentlichen 400 nm mit der Tumorzellenidentifizierungsvorrichtung (10), die eine Lichtquelle (18) und ein Sensorelement (24) umfasst, wobei die sich in der Flüssigkeit befindlichen Zellen durch die Lichtquelle (18) gesamtheitlich oder sequentiell bestrahlbar sind; und
b) gesamtheitliches oder sequentielles Erfassen der von der mindestens einen Zelle in der Flüssigkeit emittierten Lichtmenge von Licht einer Wellenlänge von im Wesentlichen 630 nm bis 700 nm durch das Sensorelement (24) und Erzeugen mindestens eines für die erfasste Lichtmenge charakteristischen Signals;
wobei die Flüssigkeit innerhalb der Tumoridentifizierungsvorrichtung (10) vor Beendigung von Schritt b) von Fremdlicht abgeschlossen gelagert wird und für das Bestrahlen der Flüssigkeit und das Erfassen der emittierten Lichtmenge gemäß den Verfahrensschritten a) und b) ein Gefäß (42) der Tumoridentifizierungsvorrichtung (10) verwendet wird, wobei das Gefäß (42) in Form einer Schale mit einem flachen Boden ausgebildet ist, in der die Flüssigkeit (40) flächig verteilbar ist und zur Lenkung des Lichts auf diesen Boden mindestens ein Spiegelelement (44) in der Tumoridentifizierungsvorrichtung (10) ausgebildet ist.

2. Tumorzellenidentifizierungsverfahren nach Anspruch 1,
**dadurch gekennzeichnet,**
**dass** die Flüssigkeit vor und/oder während und/oder nach der Durchführung von Schritt a) und/oder Schritt b) bewegt wird.

3. Tumorzeltenidentifizierungsverfahren nach Anspruch 2,
**dadurch gekennzeichnet,**
**dass** die Bewegung durch die Schwerkraft oder eine Pumpe erfolgt.

4. Tumorzellenidentifizierungsverfahren nach einem der Ansprüche 1 bis 3
**dadurch gekennzeichnet,**
**dass** die mindestens eine Zelle einen Anteil einer Zellgesamtheit in der Flüssigkeit darstellt und die Schritte a) und b) nacheinander mit sämtlichen Anteilen der Zellgesamtheit durchgeführt werden.

5. Tumorzeltenidentifizierungsverfahren nach einem der Ansprüche 1 bis 4,
**dadurch gekennzeichnet,**
**dass** das mindestens eine Signal in mindestens einem weiteren Schritt verstärkt und/oder umgewandelt und/oder mit einem vorbestimmten Protoporphyrin-IX-Konzentrationsgrenzwert verglichen wird, wobei ein Ausgabesignal erzeugt wird, das angibt, mit welcher Wahrscheinlichkeit in der Flüssigkeit mindestens eine Tumorzelle vorliegt.

6. Tumorzellenidentifizierungsverfahren nach Anspruch 1,
**dadurch gekennzeichnet,**
**dass** das Gefäß (42) aus Glas besteht, insbesondere eine Glasschale ist.

7. Tumorzellenidentifizierungsverfahren nach einem der Ansprüche 1 oder 6,
**dadurch gekennzeichnet,**
**dass** das Gefäß (42) mindestens in einem Bereich lichtdurchlässig, insbesondere für Licht der Wellenlänge 350 nm bis 450 nm und/oder von 600 nm bis 700 nm, ist.

8. Tumorzellenidentifizierungsverfahren nach einem der vorhergehenden Ansprüche,
**dadurch gekennzeichnet,**
**dass** das Gefäß (42) und/oder die Lichtquelle (18) und/oder das Sensorelement (24) und/oder das mindestens eine Spiegelelement (44) und/oder die Messkammer (12) innerhalb einer Dunkelkammer (36) angeordnet ist.

9. Tumorzellenidentifizierungsverfahren nach einem der vorhergehenden Ansprüche,
**dadurch gekennzeichnet,**
**dass** die Uchtquelle (18) mindestens einen Filter (22) aufweist, der nur für Licht einer Wellenlänge von im Wesentlichen 400 nm durchlässig ist.

10. Tumorzellenidentifizierungsverfahren nach einem der vorhergehenden Ansprüche,
**dadurch gekennzeichnet,**
**dass** das Sensorelement (24) mindestens einen Filter (26) aufweist, der nur für Licht einer Wellenlänge von im Wesentlichen 630 nm bis 700 nm durchlässig ist.

11. Tumorzellenidentifizierungsverfahren nach einem der vorhergehenden Ansprüche,
**dadurch gekennzeichnet,**
**dass** die Tumorzellenidentifizierungsvorrichtung (10) eine Ausgabeeinheit (30) aufweist, durch die das mindestens eine von dem Sensorelement (24) erzeugte Signal verstärkbar und/oder umwandelbar und/oder mit einem vorbestimmten Protoporphyrin-IX-Konzentrationsgrenzwert vergleichbar ist, wobei durch die Ausgabeeinheit (30) ein Ausgabesignal erzeugbar ist, das angibt, mit welcher Wahrscheinlichkeit in der Flüssigkeit mindestens eine Tumorzelle vorliegt.

12. Tumorzellenidentifizierungsverfahren nach einem der vorhergehenden Ansprüche,
**dadurch gekennzeichnet,**
**dass** die Flüssigkeit Zellen einer Spüllösung, die mit einem Gewebe eines Patienten in Berührung gekommen ist, und/oder Zellen einer Körperflüssigkeit, insbesondere Urin und/oder Blut und/oder Speichel und/oder Scheidensekret und/oder Spermienflüssigkeit und/oder Lungenauswurf und/oder Magensaft und/oder Bauchspeicheldrüsenflüssigkeit aufweist.

13. Tumorzellenidentifizierungsverfahren nach Anspruch 12,
**dadurch gekennzeichnet,**
**dass** die Flüssigkeit die Spüllösung und/oder die Körperflüssigkeit ist.

14. Tumorzellenidentifizierungsverfahren nach einem der vorhergehenden Ansprüche,
**dadurch gekennzeichnet,**
**dass** die Tumorzellenidentifizierungsvorrichtung (10) kompakt, insbesondere tragbar, ausgebildet ist.

15. Verfahren zur Identifizierung von Tumorzellen, das das Tumorzellenidentifizierungsverfahren nach einem der Ansprüche 1 bis 14 umfasst, wobei das Verfahren zunächst folgenden Schritt umfasst:
- Anreichern von Protoporphyrin IX in den Tumorzellen.

16. Verfahren nach Anspruch 15,
**dadurch gekennzeichnet,**
**dass** in den Tumorzellen durch 30 bis 120 minütige Inkubation mit 5-Aminolävulinat (5-ALA) in vitro, insbesondere bei 37°C, Protoporphyrin IX angereichert wird.

17. Verfahren nach einem der Ansprüche 15 oder 16,
**dadurch gekennzeichnet,**
**dass** in den Tumorzellen Protoporphyrin IX angereichert wird, indem eine Lösung, die 5-Aminolävulinat (5-ALA) aufweist, in einen Patienten instilliert wird und nach 30 bis 120 min eine oder die Flüssigkeit entnommen wird.

18. Verfahren nach einem der Ansprüche 15 bis 17,
**dadurch gekennzeichnet,**
**dass** die mindestens eine Zelle durch mindestens einen Zentrifugationsschritt konzentriert wird.

## Claims

1. Tumor cell identification method for automated in vitro identification of tumor cells, in which protoporphyrin IX is enriched, in a liquid using a tumor cell identification device (10), including the following steps:
a) irradiating at least one cell in the liquid with light of a wavelength of substantially 400 nm with the tumor cell identification device (10) including a light source (18) and a sensor element (24), wherein the cells located in the liquid can be irradiated entirely or sequentially by the light source (18); and
b) entirely or sequentially detecting the light amount of light of a wavelength of substantially 630 nm to 700 nm, emitted by the at least one cell in the liquid, by the sensor element (24), and generating at least one signal characteristic of the detected light amount;
wherein the liquid is stored shielded from outside light within the tumor cell identification device (10) before completing step b), and for irradiating the liquid and detecting the emitted light amount according to method steps a) and b), a vessel (42) of the tumor cell identification device (10) is used, wherein the vessel (42) is formed in the shape of a dish having a flat bottom, in which the liquid (40) can be distributed areally, and at least one mirror element (44) is formed in the tumor cell identification device (10) for directing the light to this bottom.

2. Tumor cell identification method according to claim 1,
**characterized in that**
the liquid is agitated before and/or during and/or after performing step a) and/or step b).

3. Tumor cell identification method according to claim 2,
**characterized in that**
the agitation is effected by gravity or by a pump.

4. Tumor cell identification method according to any one of claims 1 to 3,
**characterized in that**
the at least one cell represents a portion of a cell entirety in the liquid, and the steps a) and b) are performed sequentially with all of the portions of the cell entirety.

5. Tumor cell identification method according to any one of claims 1 to 4,
**characterized in that**
the at least one signal is amplified and/or converted and/or compared to a predetermined protoporphyrin IX concentration limit value in at least one further step, wherein an output signal is generated indicating with which probability at least one tumor cell is present in the liquid.

6. Tumor cell identification method according to claim 1,
**characterized in that**
the vessel (42) is made of glass, especially is a glass dish.

7. Tumor cell identification method according to any one of claims 1 or 6,
**characterized in that**
the vessel (42) is transparent to light, especially to light of the wavelength of 350 nm to 450 nm and/or of 600 nm to 700 nm, in at least one region.

8. Tumor cell identification method according to any one of the preceding claims,
**characterized in that**
the vessel (42) and/or the light source (18) and/or the sensor element (24) and/or the at least one mirror element (44) and/or the measurement chamber (12) is disposed within a darkroom (36).

9. Tumor cell identification method according to any one of the preceding claims,
**characterized in that**
the light source (18) has at least one filter (22), which is only transparent to light of a wavelength of substantially 400 nm.

10. Tumor cell identification method according to any one of the preceding claims,
**characterized in that**
the sensor element (24) has at least one filter (26), which is only transparent to light of a wavelength of substantially 630 nm to 700 nm.

11. Tumor cell identification method according to any one of the preceding claims,
**characterized in that**
the tumor cell identification device (10) has an output unit (30), by which the at least one signal generated by the sensor element (24) can be amplified and/or converted and/or compared to a predetermined protoporphyrin IX concentration limit value, wherein an output signal can be generated by the output unit (30), which indicates with which probability at least one tumor cell is present in the liquid.

12. Tumor cell identification method according to any one of the preceding claims,
**characterized in that**
the liquid has cells of a rinsing solution, which has contacted tissue of a patient, and/or cells of a body fluid, especially urine and/or blood and/or saliva and/or vaginal secretion and/or spermatic fluid and/or pulmonary output and/or gastric juice and/or pancreatic fluid.

13. Tumor cell identification method according to claim 12,
**characterized in that**
the liquid is the rinsing solution and/or the body fluid.

14. Tumor cell identification method according to any one of the preceding claims,
**characterized in that**
the tumor cell identification device (10) is formed in compact, especially in portable manner.

15. Method for identifying tumor cells including the tumor cell identification method according to any one of claims 1 to 14, wherein the method first includes the following step:
- enriching protoporphyrin IX in the tumor cells.

16. Method according to claim 15,
**characterized in that**
in the tumor cells, protoporphyrin IX is enriched by 30 to 120 minutes incubation with 5-aminolaevulinate (5-ALA) in vitro, especially at 37°C.

17. Method according to any one of claims 15 or 16,
**characterized in that**
in the tumor cells, protoporphyrin IX is enriched by instilling a solution having 5-aminolaevulinate (5-ALA) into a patient and removing an or the liquid after 30 to 120 min.

18. Method according to any one of claims 15 to 17,
**characterized in that**
the at least one cell is concentrated by at least one centrifugation step.

## Revendications

1. Procédé d'identification de cellules tumorales pour l'identification in vitro automatisée de cellules tumorales enrichies en protoporphyrine IX, dans un liquide, en utilisant un dispositif d'identification de cellules tumorales (10), comprenant les étapes suivantes :
a) irradiation d'au moins une cellule dans le liquide avec une lumière d'une longueur d'onde, pour l'essentiel, de 400 nm par le dispositif d'identification de cellules tumorales (10) qui comprend une source de lumière (18) et un élément détecteur (24), les cellules figurant dans le liquide pouvant être illuminées globalement ou séquentiellement par la source de lumière (18) ; et
b) acquisition globale ou séquentielle de la quantité de lumière émise par la au moins une cellule dans le liquide, d'une longueur d'onde, pour l'essentiel, de 630 nm à 700 nm, par l'élément détecteur (24) et création d'au moins un signal caractéristique de la quantité de lumière acquise ;
le liquide à l'intérieur du dispositif d'identification de cellules tumorales (10) étant stocké enfermé et protégé contre la lumière étrangère avant achèvement de l'étape b) et, pour l'irradiation du liquide et l'acquisition de la quantité de lumière émise selon les étapes de procédé a) et b), un récipient (42) du dispositif d'identification de cellules tumorales (10) étant employé, le récipient (42) ayant la forme d'une coupe avec un fond plat dans laquelle le liquide (40) peut être réparti de façon surfacique et, pour diriger la lumière vers ce fond, au moins un élément miroir (44) étant constitué dans le dispositif d'identification de cellules tumorales (10).

2. Procédé d'identification de cellules tumorales selon la revendication 1,
**caractérisé en ce que**
le liquide est déplacé avant et/ou pendant et/ou après l'exécution de l'étape a) et/ou b).

3. Procédé d'identification de cellules tumorales selon la revendication 2,
**caractérisé en ce que**
le déplacement s'effectue par gravité ou par une pompe.

4. Procédé d'identification de cellules tumorales selon l'une quelconque des revendications 1 à 3,
**caractérisé en ce que**
la au moins une cellule représente une partie d'une quantité totale de cellules dans le liquide et que les étapes a) et b) sont réalisées l'une après l'autre avec chacune des parties de la totalité des cellules.

5. Procédé d'identification de cellules tumorales selon l'une quelconque des revendications 1 à 4,
**caractérisé en ce que**
le au moins un signal, au cours d'au moins une autre étape, est amplifié et/ou converti et/ou comparé à une valeur limite prédéterminée de concentration en protoporphyrine IX, un signal de sortie étant créé qui indique la probabilité qu'au moins une cellule tumorale figure dans le liquide.

6. Procédé d'identification de cellules tumorales selon la revendication 1,
**caractérisé en ce que**
le récipient (42) est en verre, est en particulier une coupe de verre.

7. Procédé d'identification de cellules tumorales selon l'une quelconque des revendications 1 ou 6,
**caractérisé en ce que**
le récipient (42) est, au moins dans une zone, transparent à la lumière, en particulier à la lumière d'une longueur d'onde de 350 nm à 450 nm et/ou de 600 nm à 700 nm.

8. Procédé d'identification de cellules tumorales selon l'une quelconque des revendications précédentes,
**caractérisé en ce que**
le récipient (42) et/ou la source de lumière (18) et/ou l'élément détecteur (24) et/ou le au moins un élément miroir (44) et/ou la chambre de mesure (12) sont disposés dans une chambre noire (36).

9. Procédé d'identification de cellules tumorales selon l'une quelconque des revendications précédentes,
**caractérisé en ce que**
la source de lumière (18) présente au moins un filtre (22) qui n'est transparent que pour de la lumière d'une longueur d'onde, pour l'essentiel, de 400 nm.

10. Procédé d'identification de cellules tumorales selon l'une quelconque des revendications précédentes,
**caractérisé en ce que**
l'élément détecteur (24) présente au moins un filtre (26) qui n'est transparent que pour de la lumière d'une longueur d'onde, pour l'essentiel, de 630 nm à 700 nm.

11. Procédé d'identification de cellules tumorales selon l'une quelconque des revendications précédentes,
**caractérisé en ce que**
le dispositif d'identification de cellules tumorales (10) présente une unité de sortie (30) par laquelle le au moins un signal créé par l'élément détecteur (24) peut être amplifié et/ou converti et/ou comparé à une valeur limite prédéterminée de concentration en protoporphyrine IX, un signal de sortie pouvant être créé par l'unité de sortie (30) qui indique la probabilité qu'au moins une cellule tumorale figure dans le liquide.

12. Procédé d'identification de cellules tumorales selon l'une quelconque des revendications précédentes,
**caractérisé en ce que**
le liquide présente des cellules d'une solution de rinçage qui est entrée en contact avec un tissu d'un patient et/ou des cellules d'un liquide corporel, en particulier d'urine et/ou de sang et/ou de salive et/ou d'une sécrétion vaginale et/ou de liquide spermatique et/ou d'un rejet pulmonaire et/ou de liqueur stomacale et/ou de liquide pancréatique.

13. Procédé d'identification de cellules tumorales selon la revendication 12,
**caractérisé en ce que**
le liquide est la solution de rinçage et/ou le liquide corporel.

14. Procédé d'identification de cellules tumorales selon l'une quelconque des revendications précédentes,
**caractérisé en ce que**
le dispositif d'identification de cellules tumorales (10) est réalisé de façon compacte, en particulier portable.

15. Procédé d'identification de cellules tumorales qui comprend le procédé d'identification de cellules tumorales selon l'une quelconque des revendications 1 à 14, le procédé comprenant en premier lieu l'étape suivant :
- enrichissement en protoporphyrine IX dans les cellules tumorales.

16. Procédé selon la revendication 15,
**caractérisé en ce que**,
la protoporphyrine IX est enrichie dans les cellules tumorales, par une incubation de 30 à 120 minutes avec de l'acide 5-aminolévulinique (5-ALA) in vitro, en particulier à 37 °C.

17. Procédé selon l'une quelconque des revendications 15 ou 16,
**caractérisé en ce que**
la protoporphyrine IX est enrichie dans les cellules tumorales **en ce qu'**une solution présente de l'acide 5-aminolévulinique (5-ALA) est instillée dans un patient et qu'après 30 à 120 minutes un ou le liquide est prélevé.

18. Procédé selon l'une quelconque des revendications 15 à 17,
**caractérisé en ce que**
la au moins une cellule est concentrée par au moins une étape de centrifugation.
